(19) Europäisches Patentamt
European Patent Office
Office européen des brevets

(11) Veröffentlichungsnummer: **0 241 795 B1**

(12) **EUROPÄISCHE PATENTSCHRIFT**

(45) Veröffentlichungstag der Patentschrift: **11.09.91**

(51) Int. Cl.$^5$: **C07D 487/04**, B41M 5/124, B41M 5/26, //(C07D487/04, 239:00,235:00)

(21) Anmeldenummer: **87104654.6**

(22) Anmeldetag: **30.03.87**

(54) **Benzimidazolo-chinazoline.**

(30) Priorität: **12.04.86 DE 3612440**

(43) Veröffentlichungstag der Anmeldung:
**21.10.87 Patentblatt 87/43**

(45) Bekanntmachung des Hinweises auf die Patenterteilung:
**11.09.91 Patentblatt 91/37**

(84) Benannte Vertragsstaaten:
**AT CH DE ES FR GB IT LI NL**

(56) Entgegenhaltungen:
**EP-A- 0 033 716**
**EP-A- 0 091 402**
**EP-A- 0 135 469**
**EP-A- 0 145 225**

**CHEMICAL ABSTRACTS Band 88, Nr. 23, 5. Juli 1978, page 588, column 2, Zusammenfassungnr. 17009p, Columbus, Ohio, US; R.V. BHASKAR et al.: "Formationof heterocyclic rings containing nitrogen: Part XXV-Synthesis and pyrolysis of 6.6-disubstituted 5,6-dihydrobenzimidazo(1,2-c)quinazolines"**

(73) Patentinhaber: **BAYER AG**

**W-5090 Leverkusen 1 Bayerwerk(DE)**

(72) Erfinder: **Heidenreich, Holger, Dr.**
**Andreas-Gryphius-Strasse 22**
**W-5000 Köln 80(DE)**
Erfinder: **Jabs, Gert, Dr.**
**Wingensieferkamp 25**
**W-5068 Odenthal(DE)**

**EP 0 241 795 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Gegenstand der Erfindung sind Benzimidazolo-chinazoline der allgemeinen Formel

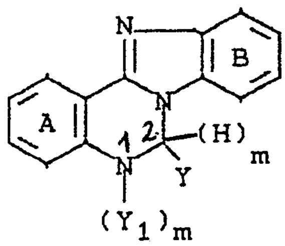

(I)

worin

Y einen Rest der Formel

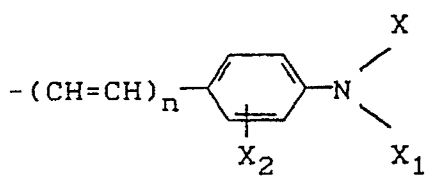

(IIa)

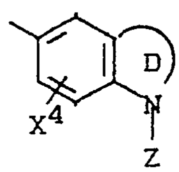

(IIb) oder

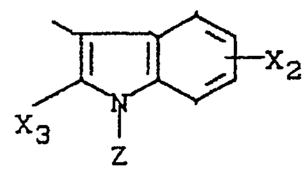

(IIc),

| | |
|---|---|
| $Y_1$ | Wasserstoff oder $C_1$- bis $C_{22}$-Alkyl |
| X und $X_1$ | unabhängig voneinander Wasserstoff, $C_1$- bis $C_{22}$-Alkyl, Cyclopentyl, Cyclohexyl, Phenyl, Napthyl, Benzyl oder Phenethyl oder |
| X und $X_1$ | zusammen mit dem sie verbindenden Stickstoff atom einen gegebenenfalls mit $C_1$- bis $C_4$-Alkyl oder Phenyl substituierten Pyrrolidin-, Pyrazolin-, Piperidin-, Piperazin- oder Morpholinrest, |
| $X_2$ | Wasserstoff, Halogen, $C_1$- bis $C_{22}$-Alkyl, $C_1$- bis $C_4$-Alkoxy oder $C_1$- bis $C_4$-Alkoxycarbonyl, |
| $X_3$ | Wasserstoff, $C_1$- bis $C_{22}$-Alkyl, Phenyl oder Naphthyl, |
| $X_4$ | Wasserstoff, Cyan, Halogen, $C_1$- bis $C_{22}$-Alkyl oder $C_1$- bis $C_4$-Alkoxy, |
| Z | Wasserstoff, $C_1$- bis $C_{22}$-Alkyl, Cyclopentyl, Cyclohexyl, Benzyl oder Phenethyl, |
| D | zusammen mit dem ankondensierten Benzolring einen Indolin-, Tetrahydrochinolin- oder Benzamorpholinrest, |

m und n unabhängig voneinander 0 oder 1 bedeuten,

worin sich zwischen den mit 1 und 2 bezeichneten Positionen, wenn m = 0, eine Doppelbindung und, wenn m = 1, eine Einfachbindung befindet, und

worin die Ringe A, B und D sowie die Reste X, $X_1$, $X_2$, $X_3$, $X_4$ und Z gegebenenfalls Halogen, Hydroxy, $C_1$- bis $C_4$-Alkoxy, Phenoxy, Naphthoxy, Benzyloxi, Phenetyloxy, Phenyl, Naphthyl, Cyclopentyl, Cyclohexyl, Pyridyl, Pyrimidyl, Pyrazinyl, Triazinyl, Imidazolyl, Oxazolyl, Thiazolyl, Triazolyl, Thiadiazolyl, Tetrazolyl, $C_1$- bis $C_{22}$-Alkylmercapto, Phenylmercapto, Naphthylmercapto, Benzylmercapto, Phenethylmercapto, $C_1$- bis $C_{22}$-Alkylsulfonyl, Cyan, $C_1$- bis $C_{22}$-Alkylcarbonyl, $C_1$-bis $C_{22}$-Alkylcarbonyloxy, Carbamoyl, $C_1$- bis $C_4$-Alkoxycarbonyl, Amino, das durch 1 oder 2 $C_1$- bis $C_{22}$-Alkyl, Phenyl, Naphthyl, Benzyl oder Phenethyl substituiert sein kann, $C_1$- bis $C_4$-Alkylcarbonylamino, $C_1$-$C_4$-Alkylsulfonylamino, Benzoylamino, $C_2$-bis $C_5$-Alkenyloxy, $C_1$- bis $C_{22}$-Alkylcarbonyloxy und Phenylcarbonyloxy, Naphthylcarbonyloxy und als Substituenten der Ringe außerdem $C_1$-bis $C_{22}$-Alkyl, Benzyl, Phenethyl, Nitro, $C_2$- bis $C_5$-Alkenyl, Phenylvinyl oder

Naphthylvinyl als Substituenten tragen können,
Verfahren zu ihrer Herstellung und ihre Verwendung als Farbbildner in druck- und wärmeempfindlichen Aufzeichnungsmaterialien.

Bevorzugt steht $C_1$-$C_{22}$-Alkyl für $C_1$-$C_{12}$-Alkyl und ganz besonders für $C_1$-$C_6$-Alkyl.

Unter Halogen ist im besonderen Fluor, Chlor und Brom zu verstehen.

Von den Verbindungen (I) sind die Benzimidazolo-chinazoline der Formel

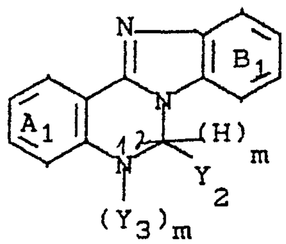

(III)

bevorzugt, worin

| | |
|---|---|
| $Y_2$ | einen Rest der Formel |

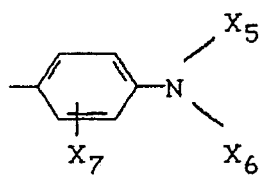

(IVa)

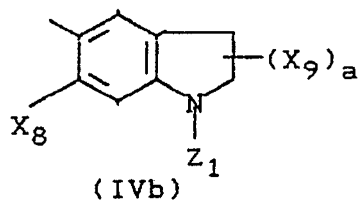

(IVb)

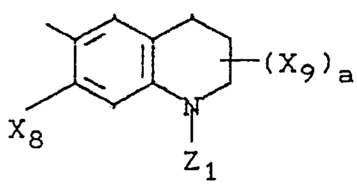

oder

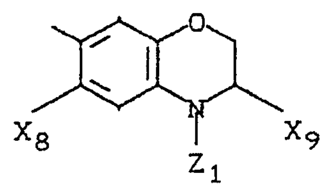

(IVc)

(IVd)

| | |
|---|---|
| $Y_3$ | Wasserstoff oder $C_1$-$C_4$-Alkyl, |
| $X_5$ und $X_6$ | unabhängig voneinander $C_1$-$C_6$-Alkyl, das durch Halogen, $C_1$-$C_4$-Alkoxy oder Cyano substituiert sein kann, Cyclopentyl oder Cyclohexyl, die durch 1-4 $C_1$-$C_4$-Alkylgruppen substituiert sein können, Phenyl, Benzyl oder Phenethyl, die durch 1 oder 2 $C_1$-$C_4$-Alkyl-,$C_1$-$C_4$-Alkoxy-, Halogen-, Nitro-, Cyano-, $C_1$-$C_4$-Alkoxycarbonyl-Reste substituiert sein können, oder |
| $X_5$ und $X_6$ | zusammen mit dem sie verbindenden Stickstoffatom einen Pyrrolidin-, Piperidin- oder Morpholinrest, die durch $C_1$-$C_4$-Alkyl substituiert sein können, |
| $X_7$ | Wasserstoff, Halogen, $C_1$-$C_4$-Alkyl, $C_1$-$C_4$-Alkoxyoder $C_1$-$C_4$-Alkoxycarbonyl, |
| $X_8$ | Wasserstoff, Halogen, $C_1$-$C_4$-Alkyl oder $C_1$-$C_4$-Alkoxy, |
| $X_9$ | $C_1$-$C_4$-Alkyl oder Phenyl oder 2 Reste $X_9$, die beide am selben C-Atom sitzen, zusammen Tri- oder Tetramethylen, |
| a | 0 oder 1 bis 4, und |
| $Z_1$ | Wasserstoff, $C_1$-$C_8$-Alkyl, das durch Halogen, Cyano oder $C_1$-$C_4$-Alkoxy substituiert sein kann, Benzyl oder Phenethyl, die durch Halogen, $C_1$-$C_4$-Alkyl oder $C_1$-$C_4$-Alkoxy substituiert sein können, bedeuten, |

worin die Ringe $A_1$ und $B_1$ durch einen oder zwei Reste ausgewählt aus Cyano, Halogen, $C_1$-$C_4$-Alkyl, $C_1$-$C_4$-Alkoxy, $NX_5X_6$, Phenyl oder Benzyl substituiert sein können, und
m die bei (I) angegebene Bedeutung hat.

Von den Verbindungen (III) sind die der Formel

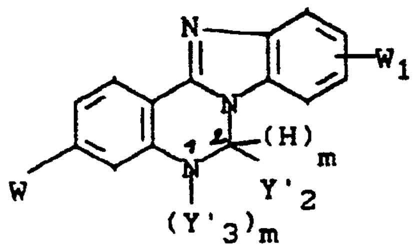

(V)

hervorzuheben, worin

W und $W_1$ Wasserstoff, Chlor, Brom, Methyl oder Methoxy,
$Y'_2$ einen Rest der Formeln

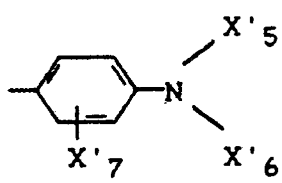

(VIa)

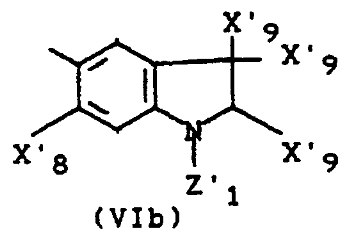

(VIb)

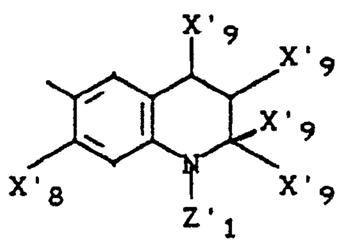

(VIc)

oder

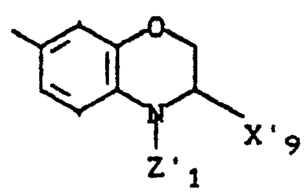

(VId)

$Y'_3$ Wasserstoff oder Methyl,
$X'_5$ und $X'_6$ unabhängig voneinander $C_1$-$C_6$-Alkyl oder Benzyl, oder
-N $X'_5$ $X'_6$ Piperidino,
$X'_7$ Wasserstoff, Methyl, Methoxy oder Ethoxy,
$X'_8$ Wasserstoff oder Methyl,
$X'_9$ Wasserstoff, Methyl oder Ethyl und
$Z'_1$ $C_1$-$C_8$-Alkyl, $\beta$-Cyanethyl oder Benzyl,
bedeuten, und m die vorstehend genannte Bedeutung hat.

Von großem Interesse sind Verbindungen der Formel

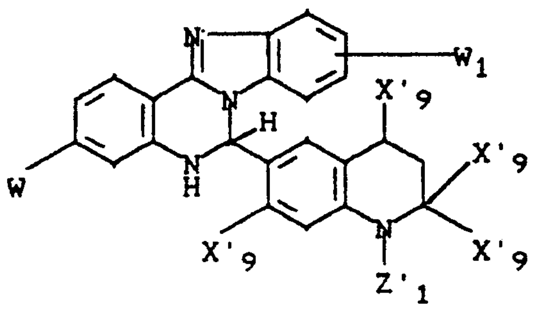

(VII)

und vor allem der Formel

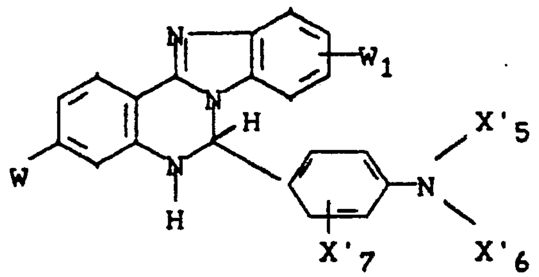

(VIII)

worin die Substituenten die vorstehend genannte Bedeutung haben.

Die erfindungsgemäßen Benzimidazolo-chinazoline der Formel (I) werden durch Umsetzung eines Aldehydes

Y-CHO (IX)

mit einer Verbindung

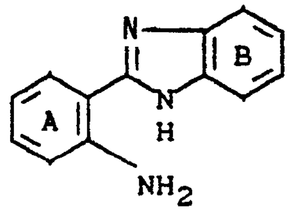

(X)

wobei A, B und Y die oben angegebene Bedeutung haben, in einem geeigneten Lösungsmittel hergestellt. Eine Doppelbindung zwischen den angezeigten Positionen 1 und 2 kann durch anschließende Oxidation erhalten werden.

Als geeignete Lösungsmittel sind niedrige aliphatische Alkohole wie Methanol, Ethanol oder Isopropanol oder cyclische Ether wie Dioxan oder Tetrahydrofuran sowie $\gamma$-Butyrolacton, Acetonitril, Dimethylsulfoxid, Dimethylformamid, Dimethylacetamid, N-Methylpyrrolidcn oder Ethylenglykolmonomethylether zu nennen.

Die Oxidation der zunächst entstehenden Dihydro-Verbindung zur Verbindung mit der Doppelbindung in Position 1 und 2 der Formel (I) wird mit Oxidationsmitteln durchgeführt, wie Chromaten, Bichromaten, Chloraten, Chloriten, Peroxiden, Mangandioxid, Bleidioxid, Chlor, Brom, molekularer Sauerstoff, Luft, Perboraten, Permanganaten, Wasserstoffperoxid und vorzugsweise Chloranil.

Die Umsetzung zur Dihydroverbindung kann bei einer Temperatur von 10° bis 150° C, vorzugsweise zwisohen 20° bis 80° C vorgenommen werden. Die Oxidation kann in Abhängigkeit vom Oxidationsmittel zwischen 10° und 150° C, vorzugsweise zwischen 20° und 100° C erfolgen.

Die Aldehyde (IX) sind literaturbekannt (z.B. "Organikum, Organisch-Chemisches Grundpraktikum, Berlin 1970, S. 355 ff.). Die Verbindungen der Formel (V) können nach D.W. Hein et al., J. Amer. Chem. Soc., Vol. 79, S. 427 (1957) erhalten werden.

Die erfindungsgemäßen Benzimidazolo-chinazoline der Formel (I) finden Verwendung als Farbbildner in druck- und wärmeempfindlichen Aufzeichnungsmaterialien. Sie sind normalerweise farblos oder höchstens schwach gefärbt.

Wenn diese Farbbildner mit einem vorzugsweise sauren Entwickler, d.h. einem Elektronenakzeptor, in Kontakt gebracht werden, so ergeben sich intensive gelbe bis rote Farbtöne, die ausgezeichnete Echtheiten aufweisen. Sie zeigen auf phenolischen Unterlagen und besonders auf aktivierten Tonen als Entwickler eine gute Farbintensität, Sublimier-und Lichtechtheit.

Typische Beispiele für Entwickler sind anorganische Stoffe wie Tone, Metallsalze oder -oxide, Phenole, Phenolcarbonsäuren bzw. deren Ester oder organische Polymerisate wie Phenolharze.

Die Verbindungen (I) können auch im Gemisch mit anderen bekannten Farbbildern, z.B. 3,3-Bis-(aminophenyl)-phthaliden, 3,3-Bis-(indolyl)-phthaliden, 3-Aminofluoranen, 2,6-Diamino-fluoranen, Leukoauraminen, Spiropyranen, Spirodipyranen, Chromenoindolen, Phenoxazinen, Phenothiazinen, Carbazolylmethanen oder weiteren Triarylmethanleukofarbstoffen eingesetzt werden.

Die Verbindungen (I) eignen sich vor allem als Farbbildner in einem Aufzeichnungsmaterial, das sowohl Kopier- als auch Registriermaterial sein kann. Ihre Entwicklungsgeschwindigkeit ist in Abhängigkeit von den Substituenten unterschiedlich. Eine geringe Entwicklungsgeschwindigkeit führt zu einer reduzierten Empfindlichkeit der Aufzeichnungsmaterialien gegenüber unbeabsichtigter vorzeitiger Entwicklung.

Insbesondere beim Thermodruck-Verfahren sind mit den erfindungsgemäßen Farbbildnern Schriftbilder extrem hoher Echtheiten und hoher Unempfindlichkeit gegen den Einfluß sowohl saurer als auch basischer Medien erhältlich.

Ein druckempfindliches Material besteht beispielsweise aus mindestens 1 Paar von Blättern, die mindestens einen Farbbildner der Formel (I), gelöst oder dispergiert in einem nichtflüchtigen organischen Lösungsmittel, und einen Elektronenakzeptor als Entwickler enthalten.

Der Farbbildner liefert an den Punkten, an deren er mit dem Elektronenakzeptor in Kontakt kommt, eine gefärbte Markierung. Um eine frühzeitige Aktivierung der Farbbildner zu verhindern, werden diese in der Regel von dem Elektronenakzeptor getrennt. Dies kann zweckmäßig erzielt werden, indem die Farbbildner in schaum-, schwamm- oder bienenwabenartigen Strukturen eingearbeitet werden. Vorzugsweise sind die Farbbildner in Mikrokapseln eingeschlossen. Verfahren zur Herstellung derartiger Mikrokapseln sind bekannt.

Als nichtflüchtige Lösungsmittel sind z.B. partiell hydriertes Terphenyl, alkylierte Naphthaline oder Dibutylphthalat geeignet.

Die Verbindungen der Formel (I) können vorzugsweise auch als Farbbilder in einem thermoreaktiven Aufzeichungsmaterial verwendet werden. Dieses enthält in der Regel mindestens einen Schichtträger, einen Farbbildner, einen Elektronenakzeptor und gegebenenfalls auch ein Bindemittel.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Meßinstrumenten, wie z.B. Elektrokardiographen, verwendet. Die Bilderzeugung (Markierung) kann auch manuell mit einer erhitzten Feder erfolgen. Eine weitere Einrichtung der Erzeugung von Markierungen mittels Wärme sind Laserstrahlen.

Als Entwickler eignen sich die gleichen Elektronenakzeptoren wie sie in druckempfindlichen Papieren verwendet werden, vorzugsweise phenolische Verbindungen, wie sie beispielsweise in der DE-PS 1 251 348 beschrieben sind, sowie Borsäure und organische, vorzugsweise aliphatische Dicarbonsäuren.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Verbindungen (I) und der Entwickler in Wasser schwerlöslich oder unlöslich sind.

Bei Einwirkung von Wärme erweicht oder schmilzt das Bindemittel, so daß der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine und Stärke.

Die thermoreaktiven Schichten können weitere Zusätze enthalten: zur Verbesserung des Weißgrades, zur Erleichterung des Bedruckens der Papiere, zur Verhinderung des Festklebens der erhitzten Feder und zur Farbbildung nur innerhalb eines begrenzten Temperaturbereiches.

Die beschriebenen Verfahren und Zubereitungen sind beispielsweise aus den US-Patentschriften 2 948 753, 3 096 189 und 3 193 404 und aus den deutschen Offenlegungsschriften 2 555 080 und 2 700 937 bekannt.

Beispiel 1

In 100 ml Ethanol erhitzt man eine Mischung aus 10,45 g 2-(o-Aminophenyl)-benzimidazol und 7,45 g 4-Dimethylaminobenzaldehyd unter Rückfluß. Nach 5 Stunden kühlt man ab und filtriert den Niederschlag ab. Man erhält 16,8 g einer Verbindung der Formel

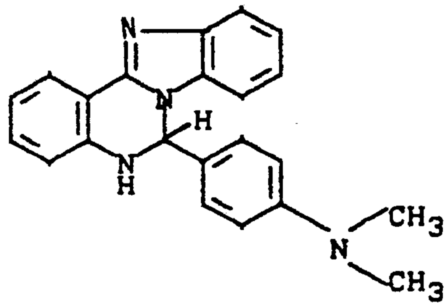

in Form schwach gelb gefärbter derber Kristalle mit dem Schmelzpunkt: 185° C.

Auf Säureton entwickelt dieser Farbbildner eine intensive gelbe Farbe.

Das oben verwendete 2-(o-Aminophenyl)-benzimidazol wird folgendermaßen erhalten: Eine Mischung aus 120 g o-Phenylendiamin und 165 g Salicylsäureethylester wird in 1 kg Polyphosphorsäure (82,5 % $P_2O_5$) langsam auf 230° C erhitzt. Man hält die Reaktionslösung 1 Stunde auf dieser Temperatur, läßt dann auf 150° C abkühlen und trägt die Lösung auf 2 kg Eis aus. Das Reaktionsprodukt wird abgesaugt, nachdem die wäßrige Lösung schwach alkalisch gestellt worden ist. Nach Umkristallisation aus Ethanol fällt ein schwach braun gefärbtes Produkt der Formel

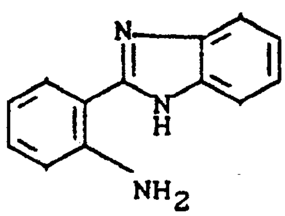

mit dem Schmelzpunkt 208° C an.

Beispiel 2

Man erhitzt eine Mischung aus 20,9 g 2-(o-Aminophenyl)-benzimidazol und 23,1 g 1,2,3,4-Tetrahydro-1-ethyl-2,2,4-trimethylchinolin-6-aldehydin 200 ml Ethanol zum Rückfluß. Nach 5 Stunden entfernt man das Lösungsmittel am Rotationsverdampfer und reibt den Rückstand mit wenig Methanol an. Man erhält 39,8 g eines fast farblosen Pulvers der Formel

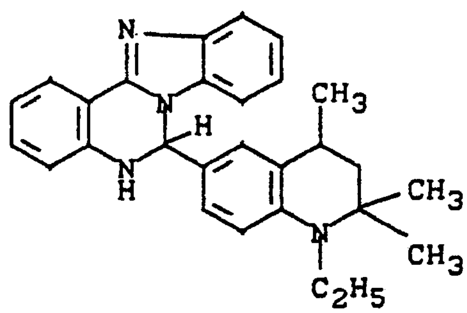

mit dem Schmelzpunkt 230° C.

Auf Säureton entwickelt dieser Farbbildner eine intensive rotstichiggelbe Farbe.

Beispiel 3

Setzt man nach dem in Beispiel 2 beschriebenen Verfahren 20,9 g 2-(o-Aminophenyl)-benzimidazol und 18,8 g 4-N-Methyl-N-Cyanethyl-benzaldehyd um, so erhält man 38,0 g eines erstarten Öls der Formel

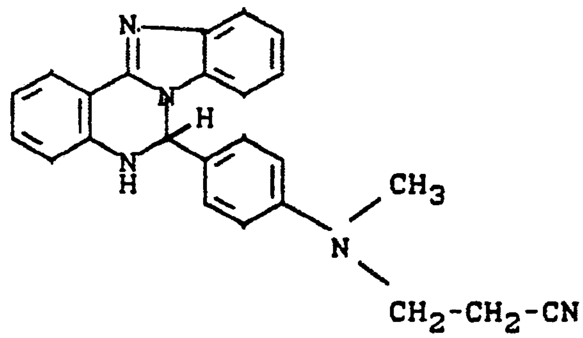

mit dem Schmelzpunkt 90 ° C.

Dieser Farbbildner entwickelt auf Säureton eine neutrale gelbe Farbe.

Beispiel 4

Läßt man 20,9 g 2-(o-Aminophenyl)-benzimidazol und 17,5 g 4-Dimethylamino-zimtaldehyd nach dem in Beispiel 2 beschriebenen Verfahren miteinander reagieren. So erhält man nach Filtration über eine Kieselgelsäule 34,5 g eines schwach braun gefärbten Öls der Formel

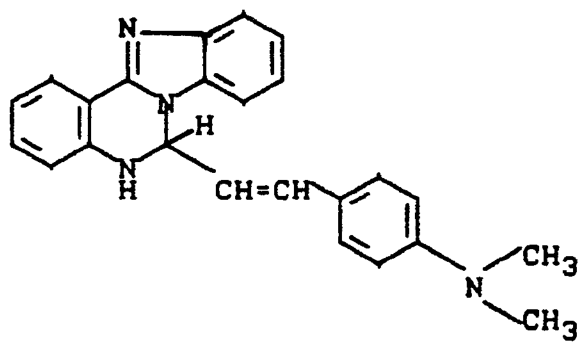

das bei 121 ° C erstarrt.

Auf Säureton liefert dieser Farbgeber eine intensive stumpfe rote Farbe.

Beispiel 5

Eine Lösung von 34,0 g des Farbgebers aus Beispiel 1 in 60 ml DMF gibt man bei einer Temperatur von 50 ° C unter starkem Rühren zu einer Suspension von 24,6 g Tetrachlorbenzochinon in 150 ml Dimethylformamid. Das Reaktionsende wird durch Dünnschichtchromatographie bestimmt. Durch Austragen auf Eis, Absaugen und Umkristallisieren aus Methanol erhält man 29,0 g fast farblose Kristalle der Formel

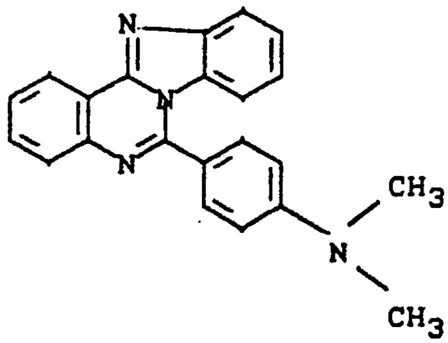

mit dem Schmelzpunkt 191 ° C.

Dieser Farbbildner entwickelt auf Säureton eine intensive gelbe Farbe.

Beispiel 6

In eine Suspension von 12,3 g Tetrachlorbenzochinon in 80 ml Dimethylformamid gibt man bei 40 ° C eine Lösung von 21,1 g des in Beispiel 2 hergestellten Farbbildners. Nach Beendigung der Oxidation (DC-Kontrolle) trägt man das Reaktionsgemisch auf Eiswasser aus, filtriert und kristallisiert den braunen Rückstand aus Methanol mit Aktivkohle um. Man isoliert 16,3 g eines schwach gelb gefärbten Pulvers der Formel

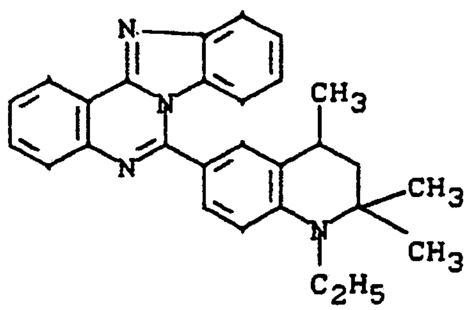

mit dem Schmelzpunkt 194 ° C.

Auf Säureton entwickelt dieser Farbbildner eine intensive rotstichiggelbe Farbe.

Nach Beispiel 1, 2 oder Beispiel 5 können auch die Farbbildner (a) und (b) hergestellt werden.

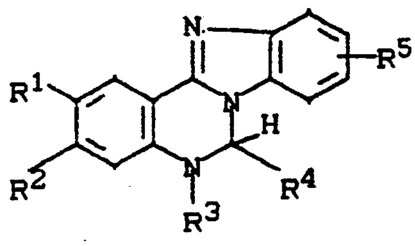

(a)

| Beispiel | $R^1$ | $R^2$ | $R^3$ | $R^4$ | $R^5$ | Farbton auf Säureton |
|---|---|---|---|---|---|---|
| 7 | H | Cl | $CH_3$ | 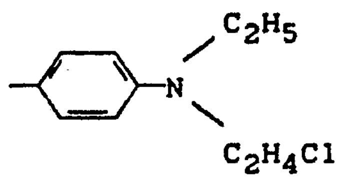 | $OCH_3$ | gelb |
| 8 | 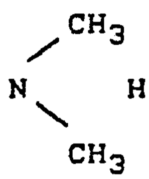 | H | H | 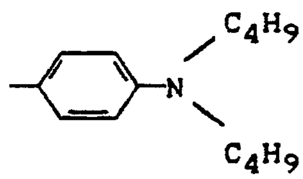 | H | rotstichig gelb |

| Beispiel | $R^1$ | $R^2$ | $R^3$ | $R^4$ | $R^5$ | Farbton auf Säureton |
|---|---|---|---|---|---|---|
| 9 | H | H | H | 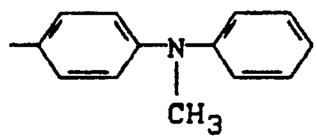 | $CH_3$ | gelb |
| 10 | H | H | H | 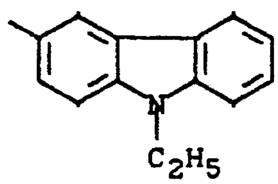 | H | gelb |
| 11 | H | Cl | $C_2H_5$ | 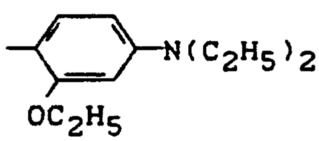 | H | rotstichig gelb |
| 12 | H | H | H | 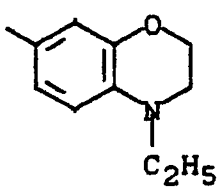 | H | rotstichig gelb |
| 13 | H | Cl | H | 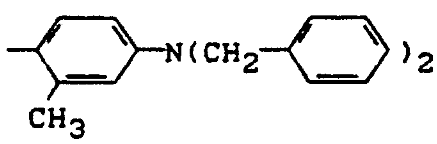 | H | gelb |
| 14 | H | H | H | 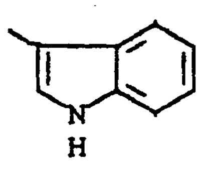 | H | gelb |

| Beispiel | $R^1$ | $R^2$ | $R^3$ | $R^4$ | $R^5$ | Farbton auf Säureton |
|---|---|---|---|---|---|---|
| 15 | H | H | H | 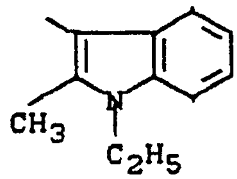 | H | gelb |
| 16 | H | H | H | 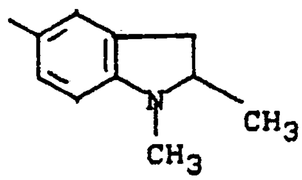 | H | gelb |
| 17 | 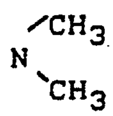 | H | H | 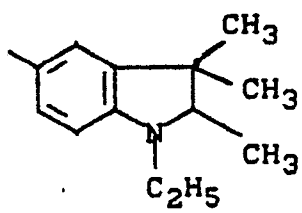 | H | rotstichig gelb |
| 18 | H | H | H | 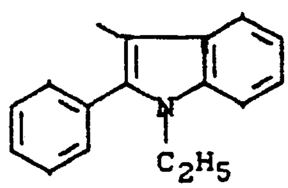 | H | gelb |
| 19 | H | H | H | 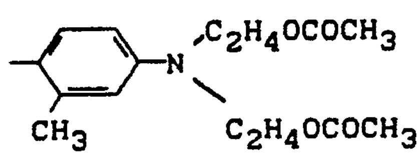 | H | gelb |

| Beispiel | $R^1$ | $R^2$ | $R^3$ | $R^4$ | $R^5$ | Farbton auf Säureton |
|---|---|---|---|---|---|---|
| 20 | H | Cl | H | 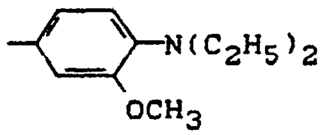 | H | rotstichig gelb |

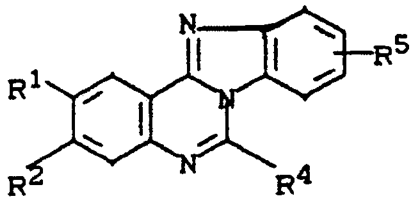

(b)

| Beispiel | $R^1$ | $R^2$ | $R^4$ | $R^5$ | Farbton |
|---|---|---|---|---|---|
| 21 | H | H | 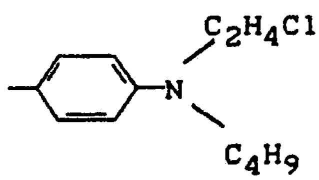 | H | gelb |
| 22 | $N(CH_3)_2$ | H | 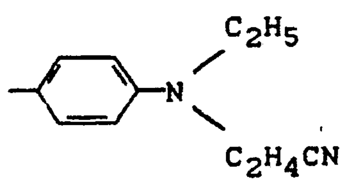 | H | gelb |
| 23 | H | Cl | 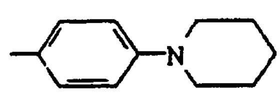 | $OCH_3$ | gelb |

| Beispiel | $R^1$ | $R^2$ | $R^4$ | $R^5$ | Farbton |
|---|---|---|---|---|---|
| 24 | H | H | 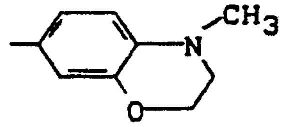 | $CH_3$ | rotstichig gelb |
| 25 | H | H | 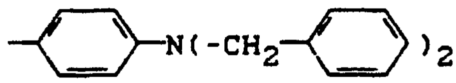 | H | gelb |
| 26 | H | H | 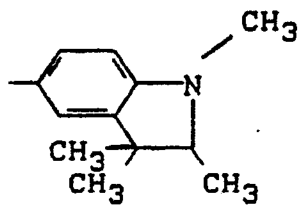 | H | gelb |
| 27 | H | H | 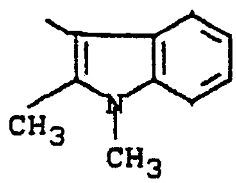 | Cl | gelb |
| 28 | H | H | 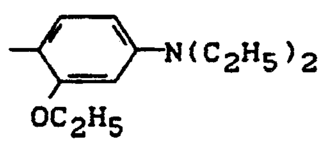 | H | rotstichig gelb |
| 29 | $N(CH_3)_2$ | H | 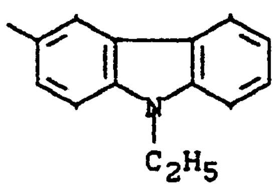 | H | rotstichig gelb |
| 30 | H | Cl | 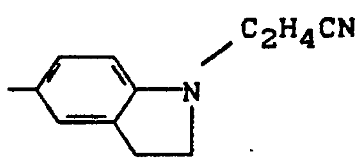 | H | gelb |

| Beispiel | $R^1$ | $R^2$ | $R^4$ | $R^5$ | Farbton |
|---|---|---|---|---|---|
| 31 | H | H | 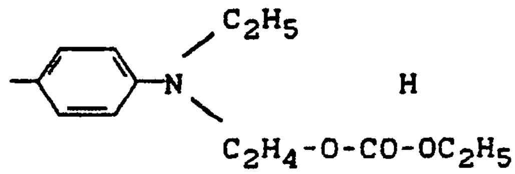 | H | gelb |

Beispiel 32

Herstellung eines kohlefreien Durchschreibepapiers

a.) Herstellung der Mikrokapseldispersion

10,0 g der Leukoverbindung, hergestellt nach Beispiel 1, werden unter Rühren und Erwärmen auf 80 °C in 161,1 g eines diisopropylierten Naphthalins gelöst und nach Abkühlen auf Raumtemperatur nacheinander 36,75 g Oxadiazintriondi-hexamethylendiisocyanat (NCO-Gehalt 21,0 %) und 40,4 g Isohexadecan unter Rühren zugefügt.

Diese organische Lösung wird in 385 g einer 0,5 %igen, wäßrigen Lösung eines teilverseiften Polyvinylacetats (Verseifungsgrad 90 %) gegeben und an eine Mischsirene bei 15 700 U/min eine Emulsion mit dieser Tröpfchengröße von 6-8 µm hergestellt. Zu dieser Emulsion werden unter Rühren 6,1 g Diethylentriamin gelöst in 61,8 g Wasser, zugefügt. Die gebildete 35 %ige Mikrokapseldispersion wird 20 Minuten bei 35 °C und nach Aufheizen 2 Stunden bei 60 °C nachgerührt.

b.) Herstellung des Durchschreibepapiers

Die in a.) hergestellte Mikrokapseldispersion wird mit Wasser auf einen Kapselanteil von 15 Gew.-% verdünnt und mit einer 30 µm Drahtrakel auf ein Rohpapier (50 g/m²) aufgestrichen.

Das Papier wird getrocknet und so ein Deckblatt eines kohlefreien Durchschreibepapiers hergestellt.

Eine Probe des Durchschreibepapiers wird mit der mit Mikrokapseln beschichteten Seite auf ein mit saurem Ton beschichtetes, handelsübliches Nehmerpapier (Giroset GF der Fa. Feldmühle) gelegt und 3 weitere Papiere aufgelegt. Mit einer Schreibmaschine wird dieser Papierset mit dem Buchstaben "w" beschriftet. Auf dem Nehmerpapier wird ein echter Durchschlag des "w" in einem intensiven gelben Farbton sichtbar.

Beispiel 33

Herstellung eines wärmeempfindlichen Aufzeichnungsblattes

In einer Kugelmühle werden 3,6 g eines Esterwachses mit einem Tropfpunkt von 79 - 85 °C (Hoechst-Wachs E der Hoechst AG), 41 g Kaolin, 18 g eines teilverseiften Polyvinylalkohols (Mowiol 26-88 der Hoechst AG), 32 g Bis-(4-hydroxyphenyl)-dimethylmethan und 500 g Wasser sorgfältig gemahlen, bis die Teilchengröße ca. 10 µm erreicht ist.

Mit Hilfe eines Ultra-Turrax-Mischers werden 10,0 g des Leukofarbstoffs, der nach Beispiel 2 hergestellt wurde, 3 g eines teilverseiften Polyvinylalkohols (Mowiol 26-88 der Hoechst AG) und 60 g Wasser bei 20.000 U/min. intensiv vermischt, bis der Farbstoff in feiner Verteilung vorliegt. Der sich dabei bildende Schaum wird durch einige Tropfen Tributylphosphat zerstört.

Die beiden Dispersionen werden miteinander vermischt und mit einer 30 µm Drahtrakel auf ein Rohpapier (50 g/m²) gestrichen und vorsichtig getrocknet. Das Beschichtungsgewicht beträgt 5,0 g/m². Wird das beschichtete Papier mit einer erhitzten Nadel berührt, entsteht eine intensive gelbe Farbe.

Entsprechende intensive, sublimier- und lichtechte gelbe bis rote Kopien werden auch bei Verwendung

jedes der anderen in den Herstellungsbeispielen 2 bis 31 angegebenen Farbbildner erzielt.

Beispiel 34

Ersetzt man in Beispiel 32 das Benzimidazolochinazolin des Beispiels 1 durch eine Mischung der folgenden Zusammensetzung:

1,4 g 3,3-Bis-(4'-dimethylaminophenyl)-6-dimethylaminophthalid,
1,0 g N-Butylcarbazol-3-yl-bis-(4'-N-methyl-N-phenylaminophenyl-)methan,
0,6 g Benzimidazolochinazolin des Beispiels 1 und
0,5 g 3,3-Bis-(N-n-octyl-2'-methylindol-3'-yl)-phthalid

und verfährt im übrigen wie in Beispiel 32 beschrieben, so erhält man ein druckempfindliches Aufzeichnungsmaterial, welches durch Schreiben mit der Hand oder mit der Schreibmaschine eine intensive und lichtechte schwarze Kopie ergibt.

## Patentansprüche

**1.** Benzimidazolo-chinazoline der allgemeinen Formel

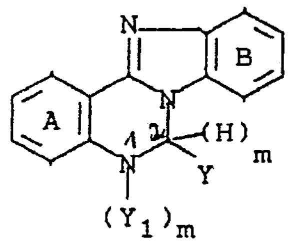

worin

Y einen Rest der Formel

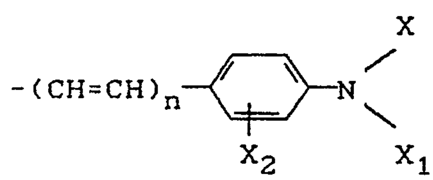

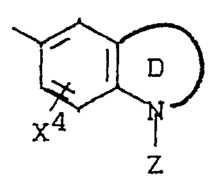

oder

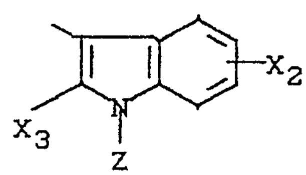

$Y_1$ Wasserstoff oder $C_1$- bis $C_{22}$-Alkyl,
X und $X_1$ unabhängig voneinander Wasserstoff, $C_1$- bis $C_{22}$-Alkyl, Cyclopentyl, Cyclohexyl, Phenyl, Naphthyl, Benzyl oder Phenethyl oder
X und $X_1$ zusammen mit dem sie verbindenden Stickstoffatom einen gegebenenfalls mit $C_1$- bis $C_4$-Alkyl oder Phenyl substituierten Pyrrolidin-, Pyrazolin-, Piperidin-, Piperazin- oder Morpholinrest,
$X_2$ Wasserstoff, Halogen, $C_1$- bis $C_{22}$-Alkyl, $C_1$- bis $C_4$-Alkoxy oder $C_1$- bis $C_4$-Alkoxycar-

bonyl,

| | |
|---|---|
| $X_3$ | Wasserstoff, $C_1$- bis $C_{22}$-Alkyl, Phenyl oder Naphthyl, |
| $X_4$ | Wasserstoff, Cyan, Halogen, $C_1$- bis $C_{22}$-Alkyl oder $C_1$- bis $C_4$-Alkoxy, |
| Z | Wasserstoff, $C_1$- bis $C_{22}$-Alkyl, Cyclopentyl, Cyclohexyl, Benzyl oder Phenethyl, |
| D | zusammen mit dem ankondensierten Benzolring einen Indolin-, Tetrahydrochinolin- oder Benzamorpholinrest, |

m und n unabhängig voneinander 0 oder 1 bedeuten,

worin sich zwischen den mit 1 und 2 bezeichneten Positionen, wenn m = 0, eine Doppelbindung und, wenn m = 1, eine Einfachbindung befindet, und

worin die Ringe A, B und D sowie die Reste X, $X_1$, $X_2$, $X_3$, $X_4$ und Z gegebenenfalls Halogen, Hydroxy, $C_1$- bis $C_4$-Alkoxy, Phenoxy, Naphthoxy, Benzyloxy, Phenethyloxy, Phenyl, Naphthyl, Cyclopentyl, Cyclohexyl, Pyridyl, Pyrimidyl, Pyrazinyl, Triazinyl, Imidazolyl, Oxazolyl, Thiazolyl, Triazolyl, Thiadiazolyl, Tetrazolyl, $C_1$-bis $C_{22}$-Alkylmercapto, Phenylmercapto, Naphthylmercapto, Benzylmercapto, Phenethylmercapto, $C_1$- bis $C_{22}$-Alkylsulfonyl, Cyan, $C_1$- bis $C_{22}$-Alkylcarbonyl, $C_1$-bis $C_{22}$-Alkylcarbonyloxy, Carbamoyl, $C_1$- bis $C_4$-Alkoxycarbonyl, Amino, das durch 1 oder 2 $C_1$- bis $C_{22}$-Alkyl, Phenyl, Naphthyl, Benzyl oder Phenethyl substituiert sein kann, $C_1$- bis $C_4$-Alkylcarbonylamino, $C_1$-$C_4$-Alkylsulfonylamino, Benzoylamino, $C_2$- bis $C_5$-Alkenyloxy, $C_1$- bis $C_{22}$-Alkylcarbonyloxy und Phenylcarbonyloxy, Naphthylcarbonyloxy und als Substituenten der Ringe außerdem $C_1$-bis $C_{22}$-Alkyl, Benzyl, Phenethyl, Nitro, $C_2$- bis $C_5$-Alkenyl, Phenylvinyl oder Naphthylvinyl als Substituenten tragen können.

2. Benzimidazolo-chinazoline gemäß Anspruch 1 der allgemeinen Formel

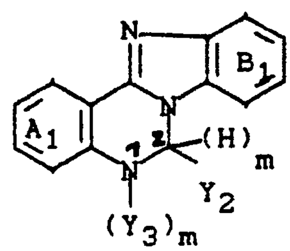

worin

$Y_2$ einen Rest der Formel

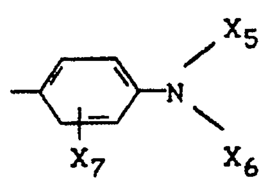

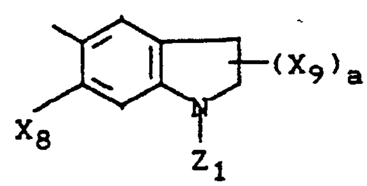

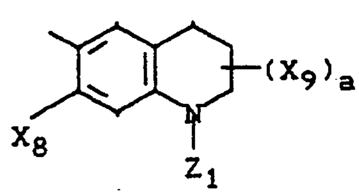

oder

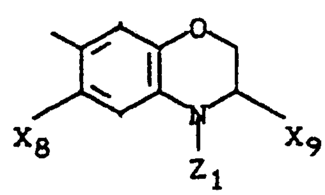

$Y_3$ Wasserstoff oder $C_1$-$C_4$-Alkyl,

$X_5$ und $X_6$ unabhängig voneinander $C_1$-$C_6$-Alkyl, das durch Halogen, $C_1$-$C_4$-Alkoxy oder Cyano substituiert sein kann, Cyclopentyl oder Cyclohexyl, die durch 1-4 $C_1$-$C_4$-Alkylgruppen substituiert sein können, Phenyl, Benzyl oder Phenethyl, die durch 1 oder 2 $C_1$-$C_4$-Alkyl-, $C_1$-$C_4$-Alkoxy-, Halogen-, Nitro-, Cyano-, $C_1$-$C_4$-Alkoxycarbonyl-Reste substituiert sein können, oder

$X_5$ und $X_6$ zusammen mit dem sie verbindenden stick-stoffatom einen Pyrrolidin-, Piperidin- oder Morpholinrest, die durch $C_1$-$C_4$-Alkyl substituiert sein können,

$X_7$ Wasserstoff, Halogen, $C_1$-$C_4$-Alkyl, $C_1$-$C_4$-Alkoxyoder $C_1$-$C_4$-Alkoxycarbonyl,

$X_8$ Wasserstoff, Halogen, $C_1$-$C_4$-Alkyl oder $C_1$-$C_4$-Alkoxy,

$X_9$ $C_1$-$C_4$-Alkyl oder Phenyl oder 2 Reste $X_9$, die beide am selben C-Atom sitzen, zusammen Tri- oder Tetramethylen,

a 0 oder 1-4, und

$Z_1$ Wasserstoff, $C_1$-$C_8$-Alkyl, das durch Halogen, Cyano oder $C_1$-$C_4$-Alkoxy substituiert sein kann, Benzyl oder Phenethyl, die durch Halogen, $C_1$-$C_4$-Alkyl oder $C_1$-$C_4$-Alkoxy substituiert sein können, bedeuten,

worin die Ringe $A_1$ und $B_1$ durch einen oder zwei Reste ausgewählt aus Cyano, Halogen, $C_1$-$C_4$-Alkyl, $C_1$-$C_4$-Alkoxy, $NX_5X_6$, Phenyl oder Benzyl substituiert sein können, und

m die in Anspruch 1 angegebene Bedeutung hat.

3. Benzimidazolo-chinazoline gemäß Anspruch 1 der allgemeinen Formel

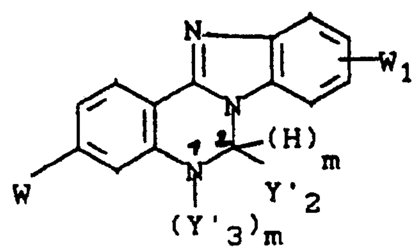

worin

W und $W_1$ Wasserstoff, Chlor, Brom, Methyl oder Methoxy,

$Y'_2$ einen Rest der Formeln

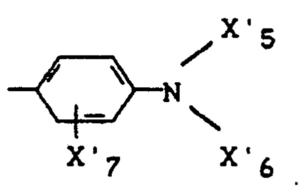

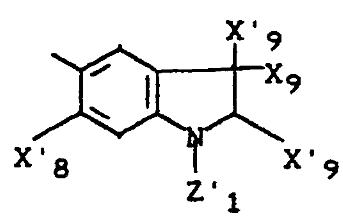

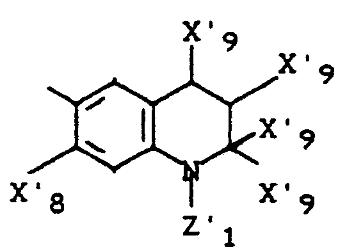

oder

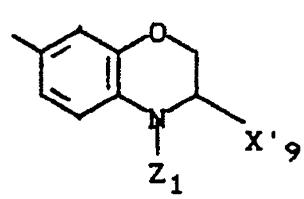

| | |
|---|---|
| $Y'_3$ | Wasserstoff oder Methyl, |
| $X'_5$ und $X'_6$ | unabhängig voneinander $C_1$-$C_6$-Alkyl oder Benzyl, oder |

-N $X'_5$ $X'_6$ Piperidino,

| | |
|---|---|
| $X'_7$ | Wasserstoff, Methyl, Methoxy oder Ethoxy, |
| $X'_8$ | Wasserstoff oder Methyl, |
| $X'_9$ | Wasserstoff, Methyl oder Ethyl und |
| $Z'_1$ | $C_1$-$C_8$-Alkyl, $\beta$-Cyanethyl oder Benzyl, |

bedeuten, und m die in Anspruch 1 genannte Bedeutung hat.

4. Benzimidazolo-chinazoline gemäß Anspruch 1 der allgemeinen Formel

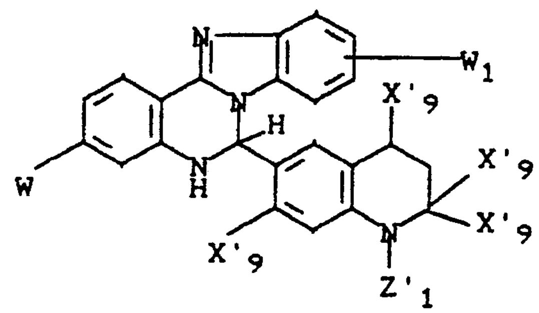

und worin die Symbole die in Anspruch 3 angegebene Bedeutung haben.

5. Benzimidazolo-chinazoline gemäß Anspruch 1 der allgemeinen Formel

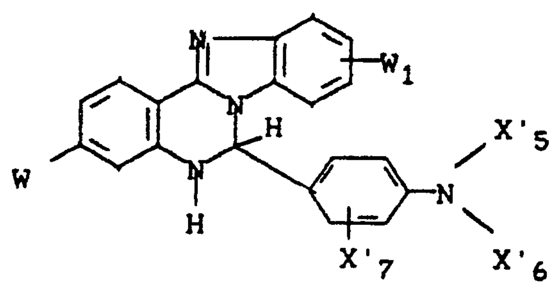

worin die Symbole die in Anspruch 3 angegebene Bedeutung haben.

6. Verfahren zur Herstellung der Benzimidazolo-chinazoline des Anspruchs 1, dadurch gekennzeichnet, daß man Aldehyde der allgemeinen Formel

Y-CHO

mit Verbindungen der allgemeinen Formel

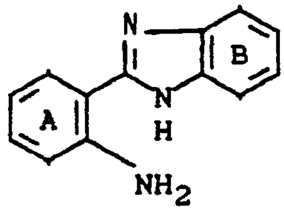

(X)

worin A, B und Y die in Anspruch 1 angegebene Bedeutung haben, umsetzt und gegebenenfalls anschließend oxydiert.

7. Verwendung der Benzimidazolo-chinazoline des Anspruchs 1 als Farbbildner in einem druck- oder wärmeempfindlichen Aufzeichnungsmaterial.

8. Druck- oder wärmeempfindliches Aufzeichnungsmaterial, dadurch gekennzeichnet, daß es in seinem Reaktantensystem mindestens eine Verbindung des Anspruchs 1 als Farbbildner enthält.

## Claims

1. Benzimidazolo-quinazolines of the general formula

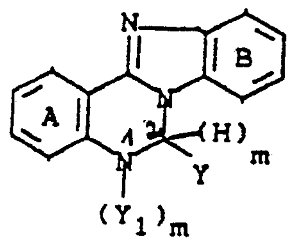

in which

Y denotes a radical of the formula

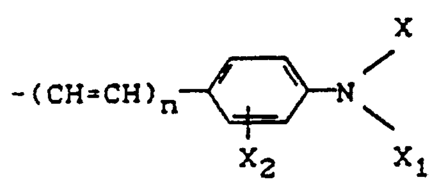

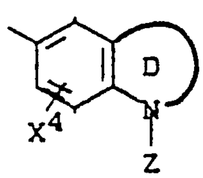

or

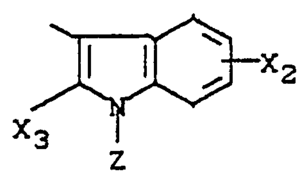

| | |
|---|---|
| $Y_1$ | denotes hydrogen or $C_1$- to $C_{22}$-alkyl, |
| X and $X_1$, | independently of one another, denote hydrogen, $C_1$- to $C_{22}$-alkyl, cyclopentyl, cyclohexyl, phenyl, naphthyl, benzyl or phenethyl or |
| X and $X_1$, | together with the nitrogen atom connecting them, denote a pyrrolidine, pyrazoline, piperidine, piperazine or morpholine radical which is optionally substituted by $C_1$- to |

$C_4$-alkyl or phenyl,

| | |
|---|---|
| $X_2$ | denotes hydrogen, halogen, $C_1$- to $C_{22}$-alkyl, $C_1$- to $C_4$-alkoxy or $C_1$- to $C_4$-alkoxycarbonyl, |
| $X_3$ | denotes hydrogen, $C_1$- to $C_{22}$-alkyl, phenyl or naphthyl, |
| $X_4$ | denotes hydrogen, cyano, halogen, $C_1$- to $C_{22}$-alkyl or $C_1$- to $C_4$-alkoxy, |
| Z | denotes hydrogen, $C_1$- to $C_{22}$-alkyl, cyclopentyl, cyclohexyl, benzyl or phenethyl, |
| D, | together with the fused benzene ring, denotes an indoline, tetrahydroquinoline or benzamorpholine radical, |

m and n, independently of one another, denote 0 or 1,

in which the positions designated 1 and 2 are connected by a double bond if m = 0 and by a single bond if m = 1, and

in which the rings A, B and D and the radicals X, $X_1$, $X_2$, $X_3$, $X_4$ and Z may optionally carry as substituents halogen, hydroxy, $C_1$- to $C_4$-alkoxy, phenoxy, naphthoxy, benzyloxy, phenethyloxy, phenyl, naphthyl, cyclopentyl, cyclohexyl, pyridyl, pyrimidyl, pyrazinyl, triazinyl, imidazolyl, oxazolyl, thiazolyl, triazolyl, thiadiazolyl, tetrazolyl, $C_1$- to $C_{22}$-alkylmercapto, phenylmercapto, naphthylmercapto, benzylmercapto, phenethylmercapto, $C_1$- to $C_{22}$-alkylsulfonyl, cyano, $C_1$- to $C_{22}$-alkylcarbonyl, $C_1$- to $C_{22}$-alkylcarbonyloxy, carbamoyl, $C_1$- to $C_4$-alkoxycarbonyl, amino which may be substituted by 1 or 2 $C_1$- to $C_{22}$-alkyl, phenyl, naphthyl, benzyl or phenethyl, $C_1$- to $C_4$-alkylcarbonylamino, $C_1$- to $C_4$-alkylsulphonylamino, benzoylamino, $C_2$-to $C_5$-alkenyloxy, $C_1$-to $C_{22}$-alkylcarbonyloxy and phenylcarbonyloxy, naphthylcarbonyloxy and in addition, as substituents of the rings, $C_1$- to $C_{22}$-alkyl, benzyl, phenethyl, nitro, $C_2$- to $C_5$-alkenyl, phenylvinyl or naphthylvinyl.

**2.** Benzimidazolo-quinazolines according to claim 1 of the general formula

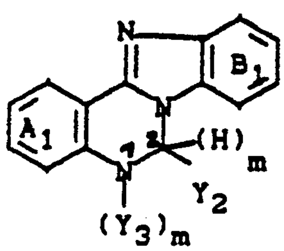

in which

$Y_2$ denotes a radical of the formula

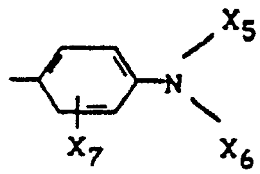
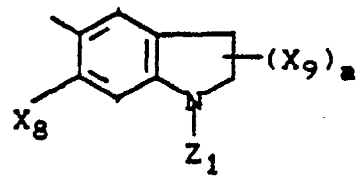

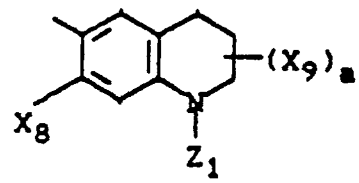

or

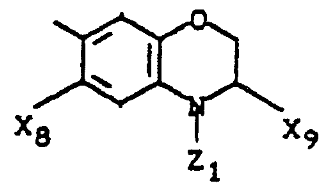

$Y_3$ denotes hydrogen or $C_1$-$C_4$-alkyl,

$X_5$ and $X_6$, independently of one another, denote $C_1$-$C_6$-alkyl which may be substituted by halogen, $C_1$-$C_4$-alkoxy or cyano, cyclopentyl or cyclohexyl, each of which may be substituted by 1-4 $C_1$-$C_4$-alkyl groups, or denote phenyl, benzyl or phenethyl, each of which may be substituted by 1 or 2 $C_1$-$C_4$-alkyl, $C_1$-$C_4$-alkoxy, halogen, nitro, cyano or $C_1$-$C_4$-alkoxycarbonyl radicals, or

$X_5$ and $X_6$, together with the nitrogen atom connecting them, denote a pyrrolidine, piperidine or morpholine radical which may be substituted by $C_1$-$C_4$-alkyl,

$X_7$ denotes hydrogen, halogen, $C_1$-$C_4$-alkyl, $C_1$-$C_4$-alkoxy or $C_1$-$C_4$-alkoxycarbonyl,

$X_8$ denotes hydrogen, halogen, $C_1$-$C_4$-alkyl or $C_1$-$C_4$-alkoxy,

$X_9$ denotes $C_1$-$C_4$-alkyl or phenyl, or 2 radicals $X_9$, which are both located on the same C atom, together denote trimethylene or tetramethylene,

a denotes 0 or 1-4, and

$Z_1$ denotes hydrogen, $C_1$-$C_8$-alkyl which may be substituted by halogen, cyano or $C_1$-$C_4$-alkoxy, benzyl or phenethyl, each of which may be substituted by halogen, $C_1$-$C_4$-alkyl or $C_1$-$C_4$-alkoxy,

in which the rings $A_1$ and $B_1$ may be substituted by one or two radicals selected from cyano, halogen, $C_1$-$C_4$-alkyl, $C_1$-$C_4$-alkoxy, $NX_5X_6$, phenyl or benzyl, and

m has the meaning specified in Claim 1.

**3.** Benzimidazolo-quinazolines according to Claim 1 of the general formula

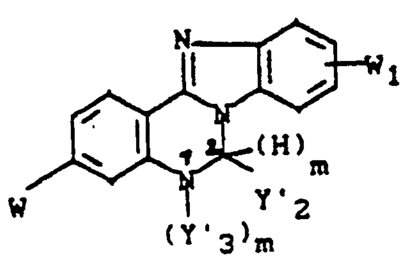

in which

W and $W_1$ denote hydrogen, chlorine, bromine, methyl or methoxy,

$Y'_2$ denotes a radical of the formulae

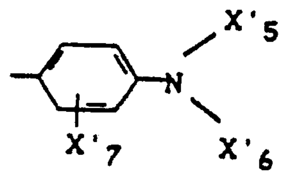

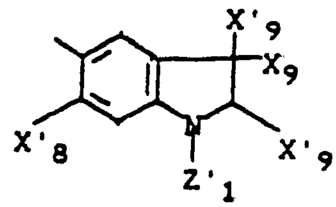

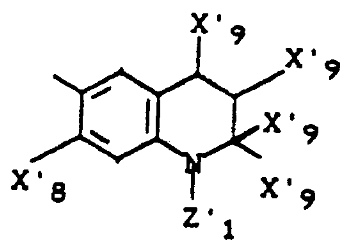

or

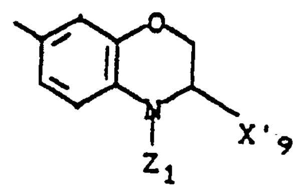

$Y'_3$ denotes hydrogen or methyl,
$X'_5$ and $x'_6$, independently of one another, denote $C_1$-$C_6$-alkyl or benzyl, or

-N $X'_5$ $X'_6$ denotes piperidino,

$X'_7$ denotes hydrogen, methyl, methoxy or ethoxy,
$X'_8$ denotes hydrogen or methyl,
$X'_9$ denotes hydrogen, methyl or ethyl, and
$Z'_1$ denotes $C_1$-$C_8$-alkyl, $\beta$-cyanoethyl or benzyl, and

m has the meaning mentioned in Claim 1.

**4.** Benzimidazolo-quinazolines according to Claim 1 of the general formula

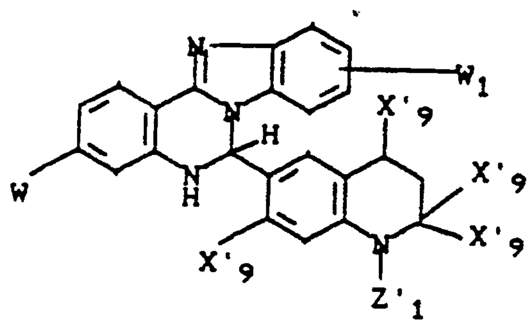

and in which the symbols have the meaning specified in Claim 3.

**5.** Benzimidazolo-quinazolines according to Claim 1 of the general formula

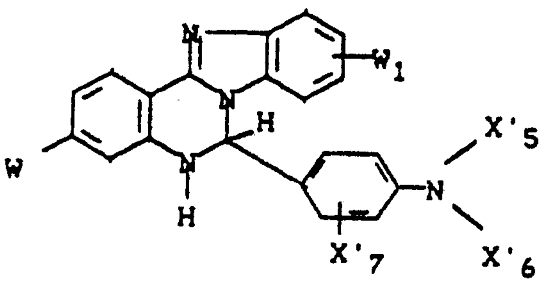

in which the symbols have the meaning specified in Claim 3.

6. Process for the preparation of the benzimidazolo-quinazolines of Claim 1, characterised in that aldehydes of the general formula

Y-CHO

are reacted with compounds of the general formula

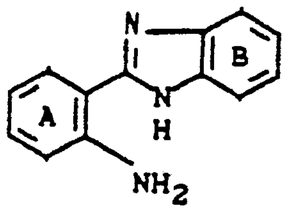

(X)

in which A, B and Y have the meaning specified in Claim 1, and, if appropriate, are subsequently oxidised.

7. Use of the benzimidazolo-quinazolines of Claim 1 as colour formers in a pressure- or heat-sensitive recording material.

8. Pressure- or heat-sensitive recording material, characterised in that it contains, in its reactant system, at least one compound of Claim 1 as colour former.

**Revendications**

1. Benzimidazolo-quinazolines de formule générale

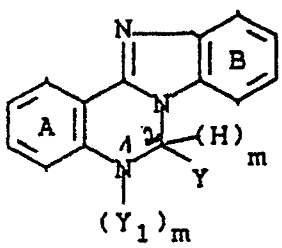

dans laquelle

Y est un reste de formule

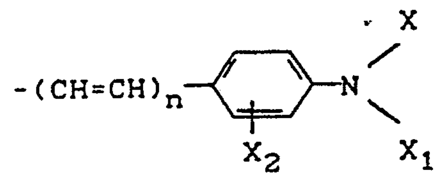

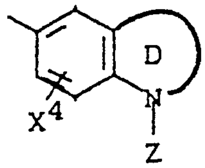

ou

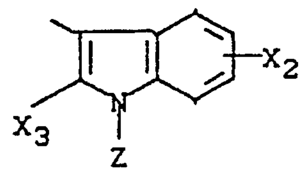

| | |
|---|---|
| $Y_1$ | est l'hydrogène ou un groupe alkyle en $C_1$ à $C_{22}$, |
| X et $X_1$ | représentent, indépendamment l'un de l'autre, l'hydrogène, un groupe alkyle en $C_1$ à $C_{22}$, cyclopentyle, cyclohexyle, phényle, naphtyle, benzyle ou phénéthyle, ou bien |
| X et $X_1$ | forment conjointement avec l'atome d'azote qui les lie, un reste pyrrolidine, pyrazoline, pipéridine, pipérazine ou morpholine éventuellement substitués avec un radical alkyle en $C_1$ à $C_4$ ou phényle, |
| $X_2$ | est l'hydrogène, un halogène, un groupe alkyle en $C_1$ à $C_{22}$, alkoxy en $C_1$ à $C_4$ ou (alkoxy en $C_1$ à $C_4$)-carbonyle, |
| $X_3$ | représente l'hydrogène, un groupe alkyle en $C_1$ à $C_{22}$, phényle ou naphtyle, |
| $X_4$ | est l'hydrogène, un groupe cyano, un halogène, un groupe alkyle en $C_1$ à $C_{22}$ ou alkoxy en $C_1$ à $C_4$, |
| Z | est l'hydrogène, un groupe alkyle en $C_1$ à $C_{22}$, cyclopentyle, cyclohexyle, benzyle ou phénéthyle, |
| D | forme conjointement avec le noyau benzénique condensé un reste indoline, tétrahydroquinoline ou benzamorpholine, |
| m et n | ont indépendamment l'un de l'autre la valeur 0 ou 1, |

lorsque m est égal à 0, une double liaison et, lorsque m est égal à 1, une liaison simple se trouvent entre les positions désignées par 1 et 2, et

les noyaux A, B et D ainsi que les restes X, $X_1$, $X_2$, $X_3$, $X_4$ et Z peuvent porter comme substituants, le cas échéant, un halogène, un groupe hydroxy, alkoxy en $C_1$ à $C_4$, phénoxy, naphtoxy, benzyloxy, phénéthyloxy, phényle, naphtyle, cyclopentyle, cyclohexyle, pyridyle, pyrimidyle, pyrazinyle, triazinyle, imidazolyle, oxazolyle, thiazolyle, triazolyle, thiadiazolyle, tétrazolyle, alkylmercapto en $C_1$ à $c_{22}$, phénylmercapto, naphtylmercapto, benzylmercapto, phénéthylmercapto, alkylsulfonyle en $C_1$ à $C_{22}$, cyano, (alkyle en $C_1$ à $C_{22}$)carbonyle, (alkyle en $C_1$ à $C_{22}$) carbonyloxy, carbamoyle, (alkoxy en $C_1$ à $C_4$)carbonyle, amino qui peut être substitué par 1 ou 2 substituants alkyle en $C_1$ à $C_{22}$, phényle, naphtyle, benzyle ou phénéthyle, un groupe (alkyle en $C_1$ à $C_4$)-carbonylamino, (alkyle en $C_1$ à $C_4$)-sulfonylamino, benzoylamino, alcényloxy en $C_2$ à $C_5$, (alkyle en $C_1$ à $C_{22}$) carbonyloxy et phénylcarbonyloxy, naphtylcarbonyloxy et en outre, comme substituants des noyaux, un groupe alkyle en $C_1$ à $C_{22}$, benzyle, phénéthyle, nitro, alcényle en $C_2$ à $C_5$, phénylvinyle ou naphtylvinyle.

**2.** Benzimidazolo-quinazolines suivant la revendication 1, de formule générale

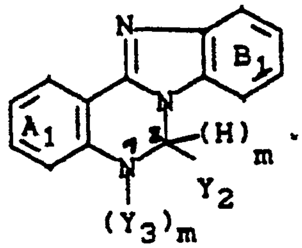

dans laquelle

$Y_2$ est un reste de formule

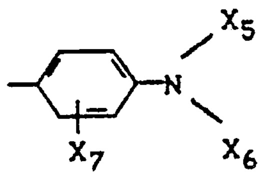

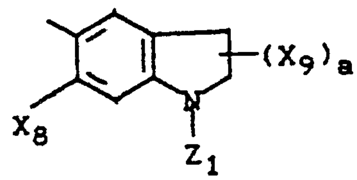

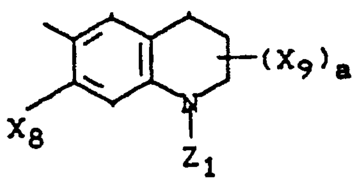

ou

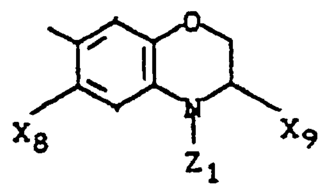

$Y_3$ est l'hydrogène ou un groupe alkyle en $C_1$ à $C_4$,

$X_5$ et $X_6$ représentent, indépendamment l'un de de l'autre, un groupe alkyle en $C_1$ à $C_6$ qui peut être substitué par un halogène, un radical alkoxy en $C_1$ à $C_4$ ou cyano, un groupe cyclopentyle ou un groupe cyclohexyle qui peut être substitué par 1 à 4 groupes alkyle en $C_1$ à $C_4$, un groupe phényle, un groupe benzyle ou un groupe phénéthyle qui peut être substitué par 1 ou 2 restes alkyle en $C_1$ à $C_4$, alkoxy en $C_1$ à $C_4$, halogéno, nitro, cyano, (alkoxy en $C_1$ à $C_4$)-carbonyle, ou bien

$X_5$ et $X_6$ forment conjointement avec l'atome d'azote qui les lie un reste pyrrolidine, pipéridine ou morpholine qui peut être substitué par un radioal alkyle en $C_1$ à $C_4$,

$X_7$ est l'hydrogène, un halogène, un groupe alkyle en $C_1$ à $C_4$, alkoxy en $C_1$ à $C_4$ ou (alkoxy en $C_1$ à $C_4$)-carbonyle,

$X_8$ est l'hydrogène, un halogène, un groupe alkyle en $C_1$ à $C_4$ ou alkoxy en $C_1$ à $C_4$,

$X_9$ est un groupe alkyle en $C_1$ à $C_4$ ou phényle, ou bien deux restes $X_9$, qui sont portés par le même atome de carbone, forment conjointement un groupe triméthylène ou tétraméthylène,

a a la valeur 0 ou une valeur de 1 à 4, et

$Z_1$ est l'hydrogène, un groupe alkyle en $C_1$ à $C_8$ qui peut être substitué par un halogène, un radical cyano ou alkoxy en $C_1$ à $C_4$, un groupe benzyle ou phénéthyle qui peut être substitué par un halogène, un radical alkyle en $C_1$ à $C_4$ ou alkoxy en $C_1$ à $C_4$,

les noyaux $A_1$ et $B_1$ pouvant être substitués par un ou deux restes choisis entre des restes cyano, halogéno, alkyle en $C_1$ à $C_4$, alkoxy en $C_1$ à $C_4$, $NX_5X_6$, phényle ou benzyle, et

m a la définition indiquée dans la revendication 1.

**3.** Benzimidazolo-quinazolines suivant la revendication 1, de formule générale

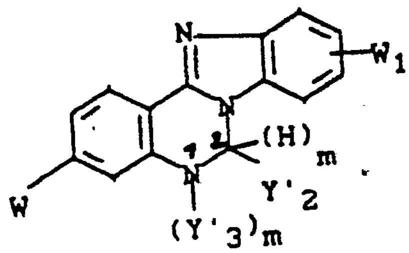

dans laquelle

W et $W_1$ représentent l'hydrogène, le chlore, le brome, un groupe méthyle ou méthoxy,

$Y'_2$ est un reste de formules

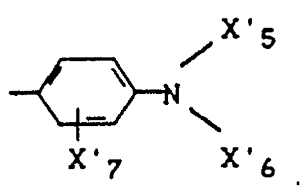

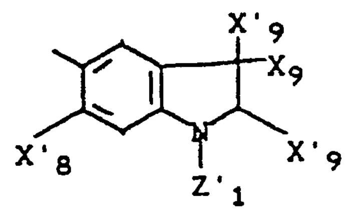

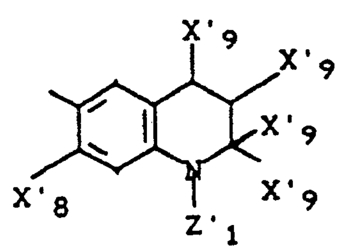

ou

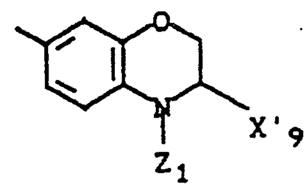

$Y'_3$ est l'hydrogène ou un groupe méthyle,

$X'_5$ et $X'_6$ représentent indépendamment l'un de l'autre un groupe alkyle en $C_1$ à $C_6$ ou benzyle, ou bien

$-NX'_5X'_6$ est un groupe pipéridino,

$X'_7$ est l'hydrogène, un groupe méthyle, méthoxy ou éthoxy,

$X'_8$ est l'hydrogène ou un groupe méthyle,

$X'_9$ est l'hydrogène, un groupe méthyle ou éthyle et

$Z'_1$ est un groupe alkyle en $C_1$ à $C_8$, $\beta$-cyanéthyle ou benzyle,

et m a la définition indiquée dans la revendication 1.

**4.** Benzimidazolo-quinazolines suivant la revendication 1, de formule générale

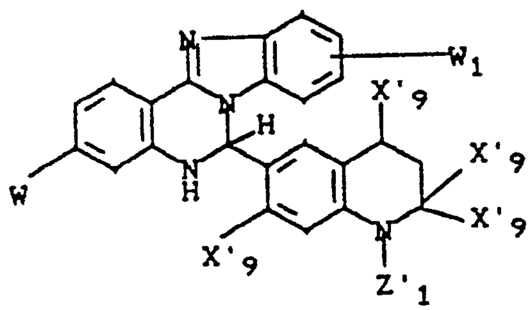

dans laquelle les symboles ont la définition indiquée dans la revendication 3.

5. Benzimidazolo-quinazolines suivant la revendication 1, de formule générale

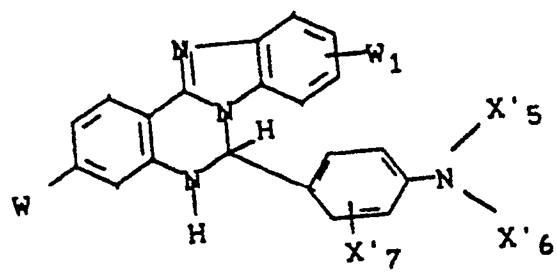

dans laquelle les symboles ont la définition indiquée dans la revendication 3.

6. Procédé de production des benzimidazoloquinazolines de formule 1, caractérisé en ce qu'on fait réagir des aldéhydes de formule générale

Y-CHO

avec des composés de formule générale

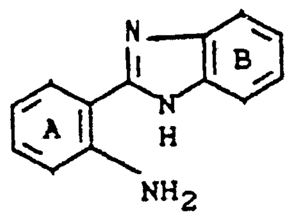

(X)

où A, B et Y ont la définition indiquée dans la revendication 1, et on les oxyde ensuite le cas échéant.

7. Utilisation des benzimidazolo-quinazolines suivant la revendication 1 comme chromogènes dans un support d'enregistrement sensible à la pression ou à la chaleur.

8. Support d'enregistrement sensible à la pression ou à la chaleur, caractérisé en ce qu'il contient comme chromogènes dans son système réactionnel au moins un composé suivant la revendication 1.